# EUROPEAN PATENT SPECIFICATION

(11) **EP 1 386 928 B1**
(45) Date of publication and mention of the grant of the patent: **14.10.2015**
(21) Application number: 02727168.3
(22) Date of filing: 08.05.2002
(51) Int. Cl.: C07K 14/435, C12N 15/12, A61K 38/00

(54) **A FROG SKIN ANTIBACTERIAL PEPTIDE DERIVATIVE**
ANTIBAKTERIELLES PEPTIDDERIVAT AUS FROSCHHAUT
DERIVE PEPTIDIQUE ANTIBACTERIEN DE PEAU DE GRENOUILLE

(30) Priority: 10.05.2001 CN 01112855
(43) Date of publication of application: 04.02.2004
(73) Proprietor: Shanghai Benemae Pharmaceutical Corporation, Zhoupu Town Pudong New Area Shanghai (CN)
(72) Inventor: SUN, Yukun, Shanghai 200233 (CN); WU, Dengxi, Shanghai 200233 (CN); ZHU, Zhiyong, Shanghai 200233 (CN); YU, Gang, Shanghai 200233 (CN); SHEN, Chunjuan, Shanghai 200233 (CN); ZHAO, Shaoling, Shanghai 200233 (CN); ZHOU, Jiaxiang, Shanghai 200233 (CN)
(74) Representative: Gulde & Partner
(86) International application number: PCT/CN2002/000317
(87) International publication number: WO 2003/016339

(56) References cited:
- US-A- 5 589 364
- US-A- 5 912 231
- TERRY A S ET AL: "THE COMPLEMENTARY DNA SEQUENCE CODING FOR PREPRO-PGS PREPROMAGAININS AND ASPECTS OF THE PROCESSING OF THIS PREPROPOLYPEPTIDE" JOURNAL OF BIOLOGICAL CHEMISTRY, vol. 263, no. 12, 1988, pages 5745-5751, XP002313365 ISSN: 0021-9258
- CHEN H-C ET AL: "SYNTHETIC MAGAININ ANALOGUES WITH IMPROVED ANTIMICROBIAL ACTIVITY" FEBS LETTERS, ELSEVIER SCIENCE PUBLISHERS, AMSTERDAM, NL, vol. 236, no. 2, 1 August 1988 (1988-08-01), pages 462-466, XP002030379 ISSN: 0014-5793
- LEE MALOY W ET AL: "STRUCTURE - ACTIVITY STUDIES ON MAGAININS AND OTHER HOST DEFENSE PEPTIDES" BIOPOLYMERS, NEW YORK, NY, US, vol. 37, 1995, pages 105-122, XP000949800 ISSN: 0006-3525
- CUERVO J H ET AL: "THE MAGAININS: SEQUENCE FACTORS RELEVANT TO INCREASED ANTIMICROBIALACTIVITY AND DECREASED HEMOLYTIC ACTIVITY" PEPTIDE RESEARCH, NATICK, MA, US, vol. 1, no. 2, November 1988 (1988-11), pages 81-86, XP002030380 ISSN: 1040-5704
- DATABASE GENBANK [Online] 28 March 1993 TERRY A.S. ET AL., XP002986617 Database accession no. (AAA49930)

### SEQUENCE LISTING

<110> Shanghai Huayi Bio Lab
<120> Derivates of Magainin
<130> USP 6183992
<150> CN 01112855.0
   <151> 2001-05-10
<160> 1
<170> PatentIn version 3.1
<210> 1
   <211> 24
   <212> PRT
   <213> Magainin analogue
<220>
   <221> VARIANT
   <222> (21)..(21)
   <223> Ile or Leu
<220>
   <221> VARIANT
   <222> (23)..(23)
   <223> Lys or Ile or Arg or Leu
<220>
   <221> VARIANT
   <222> (24)..(24)
   <223> Lys or Ile or Arg or Leu
<400> 1
   Gly Ile Gly Lys Phe Leu His Ser Ala Lys Lys Phe Gly Lys Ala Phe
   1 5 10 15
   Val Gly Glu Ile Met Asn Ser Ser
   20

## Description

### Field of the Invention

The present invention relates to derivatives of Magainin. Specifically, the present invention relates to derivatives of Magainin having the properties of anti-microbial activities.

### Background of the Invention

There are many life forms, for example, insects, microorganisms, amphibians and human beings which may produce anti-bacterial peptide materials that protect their communities. These anti-bacterial peptides can penetrate lipids on the cell membranes and make them inactive, can also affect protozoon species, germ cells and even viruses, hence such peptides are referred to as super-antibiotics. Anti-bacterial peptides all carry various amounts of positive charge, and their anti-bacterial mechanism lies in the combination of the positive charges carried by the peptides with the negative charges carried by the phospholipids which exist in the bacteria cell wall, creating an ion path on the cell membrane, enhancing the penetrability, causing the bacteria to dissolve and die. Hence the anti-microbial activities of these peptides do not depend on the binding with any specific receptors.

The anti-bacterial peptides exhibit a broad spectrum of antimicrobial activity upon gram-positive and gram-negative bacteria, as well as aerobic and anaerobic bacteria. They are different from antibiotics in that anti-bacterial peptides do not produce drug-resistance effects, even bacteria that have resistance to many types of antibiotics could be suppressed by the anti-bacterial peptides. Further, such anti-bacterial peptides also have inhibitive effect to protozoon species and viruses. As the metabolism products of the anti-bacterial peptides are amino acids, these peptides are of low toxicity for host cells. In summary, the anti-microbial peptides are a class of compounds with wide prospects of being used for anti-microbial drugs.

Magainin is a category of naturally occurring anti-bacterial peptides derived from frog skin with anti-bacterial effects. Magainin has been extensively studied up till now, they have such features as being easy to be synthesized, low in cost and little possibility of hemolysis.

US Patent USP5589364 disclosed the method by which Magainin II (23 amino acids) can be prepared using bioengineering techniques. Magainin II is a type of the naturally occurring frog-skin anti-bacterial peptide, having amino acid sequence shown as below:
Gly-Ile-Gly-Lys-Phe-Leu-His-Ser-Ala-Lys-Lys-Phe-Gly-Lys-Ala-Phe-Val-Gly-Glu-Ile-M et-Asn-Ser-OH

In which:
Gly stands for glycine, Ile for isoleucine, Lys for lysine, Phe for phenylalanine, Leu for leucine, His for histidine, Ser for serine, Ala for alanine, Val for valine, Glu for glutamic acid, Met for methionine and Asn for asparaginate.

US patent No. 6183992 disclosed a method to produce MSI-78 (22 amino acids), a derivative of magainin, having the amino acid sequence shown as below:

It has been reported in ADIS NEW DRUG PROFILE by Harriet M. Lamb, etc., that the Magainin derivative MSI-78 shows obvious curative effect in treating trauma infection and crura ulceration caused by the diabetes mellitus.

### Object of the Invention

The object of the present invention is to provide some derivates of Magainin, which broadens the category of Magainin derivatives. Such derivatives can be prepared by synthetic chemical method, and more easily, by the recombinant techniques, which makes it possible to reduce the cost of production. The anti-microbial effects of such derivatives are the same or higher than that of the naturally occurring Magainin.

### Summary of the Invention

The present invention is directed to a Magainin derivate and a pharmaceutically acceptable salt produced thereof as defined in the claims.

The Magainin derivatives of the present invention are amphoteric compounds, and may be sufficiently acidic or sufficiently basic to react with any of a number of inorganic bases, and inorganic and organic acids, to form a salt. Acids commonly employed to form acid addition salts are acids such as hydrochloric acid, hydrobromic acid, hydroiodic acid, sulfuric acid, phosphoric acid, p-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesutfonate, propanesulfonate, naphthalene-1-sulfonate, naphthalene-2-sulfonate, mandelate, and the like. Preferred acid addition salts are those formed with mineral acids such as hydrochloric acid and hydrobromic acid, and, especially, hydrochloric acid.

Alkalis may also be employed to react with the Magainin derivatives to form salts. Representative examples of such alkalis include ammonium, alkali metals, alkali metal hydroxides, carbonates and bicarbonates. Typically, such an alkali may be sodium hydroxide, potassium hydroxide, ammonium hydroxide, potassium carbonate, and the like.

The amino acids of Magainin derivatives of the present invention may be the L-typed or D-typed isomers.

One aspect of the present invention provides a method to produce the Magainin derivatives. The said method is to produce the Magainin derivatives by recombinant techniques, comprising: synthesizing gene fragments for the amino acid sequence of a Magainin derivative, ligating the gene fragments, constructing a recombinant plasmid, cloning and obtaining the product after the steps of fermentation, separation, purification and lyophilization.

It is further disclosed a method to produce the Magainin derivates.

The said method is to produce the Magainin derivates by solid phase synthesis, which comprises of using HMP resin as a solid phase carrier, protecting alpha-amine of a residue with 9-fluorenyl methoxycarbonyl (Fmoc), synthesizing the peptide on a peptide synthesizer, and obtaining products after the steps of separation, purification and lyophilization.

In still another aspect, the present invention provides the uses of such Magainin derivatives and pharmaceutically acceptable salts thereof in the preparation of drugs/pharmaceuticals having anti-microbial effects. This invention provides a variety of Magainin derivatives, broadening the category of Magainin derivatives. Such Magainin derivatives may be prepared by chemical synthesis, and more easily, by recombinant techniques, which makes it possible to decrease the production cost. Such derivatives have the same or higher anti-microbial effects compared to the naturally occurring Magainin.

It has been shown by the anti-microbial effect experiment, the time-kill study, the hemolysis and the acute toxicity tests that the anti-microbial effect on *Escherichia coil* of the Magainin derivatives of this invention is equivalent to that of the naturally occurring Magainin, and such Magainin derivatives also have inhibitory effects on *Staphylococcus aureus.* The anti-microbial experiment was conducted by testing the effects of the Magainin derivatives of the present invention to 50 µl of the bacterial solution (10⁶ bacteria/ml), more than 90% of the bacteria were killed within three hours with the dosage of 0.5 µg, while with the dosage of 1 µg, almost all the bacteria were killed within three hours. The hemolysis studies of the derivatives of the present invention showed that the ratio of the effective concentrations for hemolysis of 50% of the bacteria (HC₅₀) to inhibition of 50% of the bacteria (IC₅₀) was about 50:1, while the ratio of the naturally occurring Magainin was about 24:1, which demonstrates the safety of the products of this invention is superior to that of the naturally occurring Magainin. Further, the acute toxicity studies showed the Magainin derivatives of the present invention are of low toxicity.

### Description of the Figures

The present invention will be further illustrated with reference to the following figures and examples.

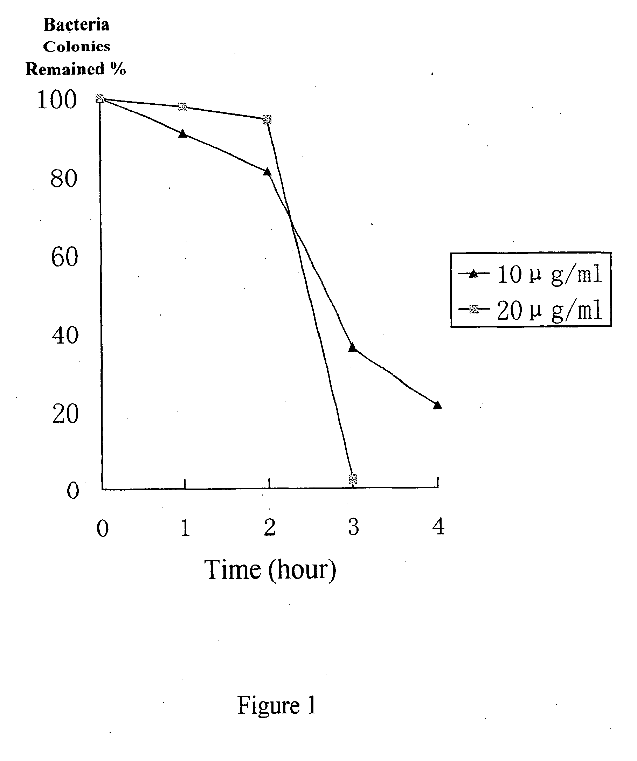

Figure 1 is the diagram showing the time-kill study of the Magainin derivative obtained from example 1 on *Escherichia coli.*

### Description of the Invention

The following examples are illustrative and are not intended to limit the scope of the present invention by any means.

### Example 1

Preparation of the Magainin derivative of the present invention by bioengineering techniques wherein X is Leu and Y is Ser-Arg
(1) Synthesizing the following gene fragments by the amino acid sequence of the above Magainin derivative:
   (a) 5'- AAT TCC ATG GGT ATC GGT AAA TTT CTG CAC AGC GCG AAA AAA
   (b) 5'- TTT GGT AAA GCG TTT GTG GGT GAA ATC CTG AAC AGC CGT TAG A
   (c)5'-AG CTT CTA ACG GCT GTT CAG GaT TTC ACC CAC AAA CGC TTT
   (d) 5'- ACC AAA TTT TTT CGC GCT GTG CAG AAA TTT ACC GAT ACC CAT GG
(2) Ligation of DNA fragments:
   Fragment (a), (b), (c) and (d) with their optical density at 260nm(A₂₆₀ₙₘ) equaling to 0.1 were taken, and fragment (a) and (d), as well as fragment (b) and (c) were drawn into two tubes separately. The polynucleotide kinase buffer, polynucleotide kinase and ATPs were added to the two tubes respectively. The reaction mixture was incubated at 37°C for 60 minutes, then incubated in a water bath of 95 °C for 10 minutes, and then was naturally cooled down to room temperature. The T4 ligase buffer and the ATP solution were added, followed by the addition of the T4 ligase, the mixture was incubated overnight at 15°C for completion of the fragment ligation.
(3) Cloning
   Plasmids containing the PL promotor (or Lac, or Tac) were digested with restriction endonucleases EcoRI and HindIII, extracted with hydroxybenzene: chloroform solvent, washed for three times with chloroform, precipitated with isopropanol solvent and collected by centrifugation.
   The digested plasmids were ligated with the Magainin derived fragments, and the plasmids containing the Magainin derived gene fragments were obtained. Such plasmids were transformed into E. coli JM103 or JM 109 host cells. Bacterial colonies were screened after cultivation by agar plate.
(4) Examination
   The plasmid containing the gene fragment of the Magainin derivative was extracted from mono-cloned bacteria, and was double digested with EcoRI and HindIII. The electrophoresis was conducted on a 1% agar gel, followed by dying with ethidum bromide. The cloned gene fragment was examined by comparison with the marker, and further tested by DNA sequence analysis.
(5) Fermentation:
   The bacterial strain was incubated in a shaking bottle with the capability of 1 liter (10 bottles in total), each containing 300 ml of LB liquid media consisted of 10g of peptone, 5g of yeast extract, 5g/L of sodium chloride. 0.2 mM of Isopropyl beta-D-Thiogalactopyranoside (IPTG) was added at 37°C for the induction of the protein to be expressed. The bacterial cells were incubated overnight and harvested by centrifugation. When using the plasmid with the temperature-controlled promotor PL, the bacterial cells were cultured at 30°C for eight hours. Then the temperature of the media was increased to 42°C, and the bacterial cells were maintained for four hours to make the gene expressed.
(6) The bacterial cell walls were broken up under the effect of lysozyme at 37°C for an hour. The precipitate was treated with 6M of guanidine hydrochloride. After centrifugation, dialysis and further centrifugation steps, the inclusion bodies of protein were obtained. The inclusion bodies were washed three times, with the wash solution containing 1% sodium chloride, 0.1 % Triton X-100 and Tris-HCL buffer (20 mM, pH8). The fusion protein was identified through polyacrylamide gel electrophoresis (PAGE) containing 12% sodium dodecanesulphonate (SDS).
(7) The inclusion bodies were dissolved in 8M of carbamide solution. Under the existence of 50mM of hydrochloric acid, cyanogen bromide was added for the lysis of inclusion bodies. The solution was stirred with the protection of nitrogen and shunning of the light. After completion of the lysis reaction, crude product of the Magainin derivative was obtained through Sephadex G-25 with fast protein liquid chromatography (FPLC, AKTA™ manufactured by Amersham Pharmacia Biotech), and final product of the Magainin derivative was acquired through purification with high performance liquid chromatography (HPLC, C₁₈ column) and gradient elution with CH₃CN/0.1% TFA buffer. The HPLC analysis result of the obtained product is consistent with those products prepared by chemical synthesis.

### Example 2

Preparation of another Magainin derivative by bioengineering techniques wherein X is Leu and Y is Ser-Lys.

A. Synthesizing the following gene fragments by the amino acid sequence of the above Magainin derivative:
(a)5' AAT TCC ATG GGT ATC GGT AAA TTT CTG CAC AGC GCG AAA AAA
(b) 5' TTT GGT AAA GCG TTT GTG GGT GAA ATC CTG AAC AGC AAG TAG A
(c) 5' AG CTT CTA CTT GCT GTT CAG GAT TTC ACC CAC AAA CGC TTT
(d) (d)5' ACC AAA TTT TTT CGC GCT GTG CAG AAA TTT ACC GAT ACC CAT GG

The steps (2)-(7) of this example are the same as that of example 1, and the HPLC analysis result of the obtained Magainin derivative is consistent with those results prepared by chemical synthesis.

### Example 3

Preparation of another Magainin derivative by solid-phase synthesis wherein X is Leu and Y is Lys-Arg.

### (1) Amino acid monomers:

| | |
|---|---|
| Fmoc-L-Ala-OH | Fmoc-L-Lys(Boc)-OH |
| Fmoc-L-Asn(Trt)-OH | Fmoc-L-Met-OH |
| Fmoc-L-Asp(OtBu)-OH | Fmoc-L-Phe-OH |
| Fmoc-L-Gln(Trt)-OH | Fmoc-L-Pro-OH |
| Fmo c-L-Glu(OtBu)-OH | Fmoc-L-Ser(tBu)-OH |
| Fmoc-L-Gly-OH | Fmoc-L-Thr(tBu)-OH |
| Fmoc-L-His(Trt)-OH | Fmoc-L-TT-OH |
| Fmoc-L-Ile-OH | Fmoc-L-Tyr(tBu)-OH |
| Fmoc-L-Leu-OH | Fmoc-L-Val-OH |

In which:
Fmoc stands for 9-fluorenyl methoxycarbonyl
BOC for tert-butyloxycarbonyl
Trt for trityl
OtBu for tertiary butyl ester, and
TBu for tert-butyl.

### (2) Apparatus and Reagents:

Apparatus: Model 433A peptide synthesizer (Applied Biosystem, US)
Reagents:
   N-methyl ketopyrrolidine, methylene chloride, hexahydropyridine, methanol, dimethylaminopyridine/ DMF N, N-diisopropylethylamine/NMP, 100 mmole HBTU / 0.5 M HOBT in DMF, N, N-Dicyclohexylcarbodiimide/NMP
In which:
DMF stands for N, N-Dimethylformamide
NMP for N-methylpyrrolidone
HOBT for 1-Hydroxybenzotriazole, and
HBTU for 2-(1H-benzotriazole-yl-1,1,3,3-tetramethyl-Uronium hexafluorophosphate)

### (3) Procedures:

### a. Synthesis

Take the synthesis scale of 0.25 mmol for example, the synthesis process was described as follows. 0.25g of HMP resin was weighed and placed in a reactor vessel of the synthesizer. 1 mmol of various residues, each coupled with protecting groups, were weighed and arrayed in the synthesizer by the amino acid sequence of the insulinotropic peptide derivate from the carboxy terminal to the amino terminal. At room temperature of 25°C, reactions for removing Fmoc protection, activating a residue and attaching the activated residue to HMP resin were automatically performed under the control of a computer program. Such reactions were circulated until the whole peptide was synthesized. After completion of the synthesis, the residue-attached resin, with each residue coupled with side chain protecting groups, was air dried on a peptide synthesizer and then weighed.

### b. Removal of protecting groups and detachment of resin:

The residue-attached resin, with each residue of the insulinotropic peptide derivative coupled with protecting groups, was placed in a plugged erlenmeyer flask, and followed by addition of cleavage reagents as shown below.

| Reagent | Dosage |
|---|---|
| Water | 0.50 ml |
| Methyl phenate | 0.50 ml |
| Phenol | 0.75 g |
| Mercaptoethanol | 0.20 ml |
| trifluoroacetic acid | 10.0 ml |

The electromagnetic stirring reaction was carried out at constant temperature of 30 °C for 6 hours. After filtration step, the aqueous filtrate was collected. The resin was washed with small amount of trifluoroacetic acid. Then the collected aqueous filtrate and the washing solution were mixed together, and ether was added for precipitation. The mixture was filtrated, and the resulted precipitate was washed with small amount of ether. After evaporation in a dehumidifier, the crude product was obtained.

### c. Purification by HPLC and lyophilization

Separation and purification of the crude product was achieved by using preparative HPLC. Final product was obtained after the steps of freezing and lyophilization. Through joint analysis of chromatogram and mass spectrogram, the molecular weight of the derivative was found to be consistent with the theoretical value.

### Example 4

### Pharmacodynamic Studies

The anti-microbial experiment of the Magainin derivative obtained from Example 1 was conducted according to the following procedures, with the comparison of that of the naturally occurring Magainin.

The tested strains of Escherichia coli JM103 and staphylococcus aureus were cultured separately, and diluted to 1 X 10⁶ bacteria/ ml. 20 mM of the sterilized Tris-HCl buffer (pH6.5) was added. Then the Magainin derivative and the naturally occurring Magainin II with various concentrations were added respectively, and incubated at 37°C for different time limit. 50 µl of the cultures was taken and spread on an agar plate, and incubated at 37°C overnight. The remaining bacteria colonies were accounted for, and the percent of the killed bacteria was calculated.
(1) Table showing the anti-microbial effects:
Comparison of the anti-microbial effects between the Magainin derivative obtained from Example 1 and the naturally occurring Magainin II

| Sample | Concentration µg/ml | Bacteria colonies remained | | | | | | Bacteria killed % | |
|---|---|---|---|---|---|---|---|---|---|
| | | 0hr | | 3hr | | 4hr | | 3hr | 4hr |
| Magainin derivative obtained from Example I | 0 | 407 | 228 | | | | | | |
| | | 317 | | | | | | | |
| | 10 | | | 109 | 73 | 160 | 195 | 71.3 | 44.2 |
| | | | | 91 | | 177 | | | |
| | 20 | | | 9 | 5 | 2 | 5 | 97.8 | 99.1 |
| | | | | 7 | | 3 | | | |
| Naturally occurring Magainin II | 10 | | | 99 | 57 | 52 | 44 | 75.4 | 84.9 |
| | | | | 78 | | 48 | | | |
| | 20 | | | 12 | 4 | 6 | 4 | 97.5 | 99.2 |
| | | | | 8 | | 5 | | | |

Table showing the anti-microbial effect of the Magainin derivative obtained from Example 1 on Staphylococos anreus

| Concentration µg/ml | Bacteria colonies remained | | | Bacteria killed within two hours % |
|---|---|---|---|---|
| | 0hr | 1hr | 2hr | |
| 0 | 267 | | | |
| | 225 | | | |
| 50 | 158 | 18 | 2 | 99 |
| | 185 | 18 | 2 | 99 |
| | 172 | 18 | 2 | 99 |

(2) Figure showing the result of time-kill study: The result of time-kill study for the Magainin derivative obtained from Example 1 was referred to in Figure 1.
(3) Table showing the results of hemolysis test:

| Group | | Dosage (µg/ml) | Hemolysis % |
|---|---|---|---|
| Test Group | Naturally-occurring Magainin | 100 | 0 |
| | | 500 | 0 |
| | | 1000 | 1.9 |
| | Magainin derivative obtained from Example 1 | 100 | 0 |
| | | 500 | 0 |
| | | 1000 | 2 |
| Control Group | Triton-100 | 0.1% | 100 |

(4) Acute toxicity test: Acute toxicity test was conducted on two mice of Kunming species. The mice were injected abdominally with the Magainin derivative obtained from Example, and the livability of the two mice were observed after receiving injection for one hour.

| Dosage of abdominal injection | Livability |
|---|---|
| 100µg | 100% |
| 200µg | 100% |
| 1000µg | 100% |

## Claims

1. A Magainin derivative and pharmaceutically acceptable salts produced thereof, wherein the said derivative having the amino acid sequence of Gly-Ile-Gly-Lys-Phe-Leu-His-Ser-Ala-Lys-Lys-Phe-Gly-Lys-Ala-Phe-Val-Gly-Glu-Ile-X-Asn-Ser-Arg, wherein X is an amino acid residue selected from the group consisting of Ile and Leu.

2. The Magainin derivative and pharmaceutically acceptable salts produced thereof of claim 1, wherein the said derivative having the amino acid sequence of Gly-Ile-Gly-Lys-Phe-Leu-His-Ser-Ala-Lys-Lys-Phe-Gly-Lys-Ala-Phe-Val-Gly-Glu-Ile-Leu-Asn-Ser-Arg.

3. A method to produce the Magainin derivative as described in claim 1 or 2 by bioengineering techniques, comprising:
a synthesis of gene fragments for the amino acid sequence of the Magainin derivative, ligation of the gene fragments, construction and cloning of a recombinant plasmid, and obtaining the product after the steps of fermentation, separation, purification and lyophilization.

4. Use of the Magainin derivative and pharmaceutically acceptable salts produced thereof as described in claim 1 or 2 in the preparation of drugs having anti-microbial effects.

## Patentansprüche

1. Magainin-Derivat und daraus hergestellte, pharmazeutisch akzeptable Salze, wobei das Derivat die Aminosäuresequenz Gly-Ile-Gly-Lys-Phe-Leu-His-Ser-Ala-Lys-Lys-Phe-Gly-Lys-Ala-Phe-Val-Gly-Glu-Ile-X-Asn-Ser-Arg aufweist, wobei X ein aus der Gruppe bestehend aus Ile und Leu ausgewählter Aminosäurerest ist.

2. Magainin-Derivat und daraus hergestellte, pharmazeutisch akzeptable Salze nach Anspruch 1, wobei das Derivat die Aminosäuresequenz Gly-Ile-Gly-Lys-Phe-Leu-His-Ser-Ala-Lys-Lys-Phe-Gly-Lys-Ala-Phe-Val-Gly-Glu-Ile-Leu-Asn-Ser-Arg aufweist.

3. Verfahren zur Herstellung des Magainin-Derivats wie in Anspruch 1 oder 2 beschrieben durch Bioengineering-Techniken, umfassend:
eine Synthese von Genfragmenten für die Aminosäuresequenz des Magainin-Derivats, Ligation der Genfragmente, Aufbau und Klonen eines rekombinanten Plasmids und Erhalten des Produkts nach den Schritten Fermentation, Abtrennung, Reinigung und Gefriertrocknung.

4. Verwendung des Magainin-Derivats und daraus hergestellter, pharmazeutisch akzeptabler Salze wie in Anspruch 1 oder 2 beschrieben bei der Herstellung von antimikrobielle Wirkungen aufweisenden Arzneimitteln.

## Revendications

1. Dérivé de magainine et sels pharmaceutiquement acceptables produits à partir de ce dérivé, dans lequel ledit dérivé a la séquence d'acides aminés Gly-Ile-Gly-Lys-Phe-Leu-His-Ser-Ala-Lys-Lys-Phe-Gly-Lys-Ala-Phe-Val-Gly-Glu-Ile-X-Asn-Ser-Arg, où X est un résidu d'acides aminés sélectionnés dans le groupe constitué de Ile et Leu.

2. Dérivé de magainine et sels pharmaceutiquement acceptables produits à partir de ce dérivé selon la revendication 1, dans lequel ledit dérivé a la séquence d'acides aminés Gly-Ile-Gly-Lys-Phe-Leu-His-Ser-Ala-Lys-Lys-Phe-Gly-Lys-Ala-Phe-Val-Gly-Glu-Ile-Leu-Asn-Ser-Arg.

3. Procédé de production du dérivé de magainine selon la description de la revendication 1 ou 2 par des techniques de bio-ingénierie, comprenant:
une synthèse de fragments de gène pour la séquence d'acides aminés du dérivé de magainine, la ligature des fragments de gène, la construction et le clonage d'un plasmide recombinant, et l'obtention du produit après les étapes de fermentation, séparation, purification et lyophilisation.

4. Utilisation du dérivé de magainine et des sels pharmaceutiquement acceptables produits à partir de ce dérivé selon la description de la revendication 1 ou 2 dans la préparation de médicaments ayant des effets antimicrobiens.
